# EUROPÄISCHE PATENTSCHRIFT

(11) **EP 0 787 480 B1**
(45) Veröffentlichungstag und Bekanntmachung des Hinweises auf die Patenterteilung: **23.04.2003**
(21) Anmeldenummer: 97101994.8
(22) Anmeldetag: 07.02.1997
(51) Int. Cl.: A61K 7/06, A61K 7/48, A61K 7/043

(54) **Kosmetische Mittel, die sulfonatgruppentragende Polyamide enthalten**
Cosmetic composition containing sulfonated polyamides
Composition cosmétique contenant des polyamides sulfonatés

(30) Priorität: 12.02.1996 DE 19605076
(43) Veröffentlichungstag der Anmeldung: 06.08.1997
(73) Patentinhaber: BASF AKTIENGESELLSCHAFT, 67056 Ludwigshafen (DE)
(72) Erfinder: Müller, Wolfgang, Dr., D-67227 Frankenthal (DE); Sanner, Axel, Dr., D-67227 Frankenthal (DE); Stein, Stefan, Dr., D-55291 Saulheim (DE); Sperling, Karin, Dr., D-67433 Neustadt (DE); Breitenbach, Jörg, Dr., D-68199 Mannheim (DE)
(74) Vertreter: Kinzebach, Werner, Dr.

(56) Entgegenhaltungen:
- EP-A- 0 696 607

## Beschreibung

Die vorliegende Erfindung betrifft die Verwendung von sulfonatgruppentragenden Polyamiden zur Behandlung von keratinhaltigen Strukturen und kosmetische Mittel, welche diese Polyamide enthalten.

Zum Festigen, Strukturverbessern und Formgeben von keratinhaltigen Strukturen werden im allgemeinen synthetische Polymere verwendet. Bewährte Polymere sind beispielsweise solche auf Basis von Vinylpyrrolidon und Vinylacetat, die üblicherweise in Form alkoholischer oder wäßrig-alkoholischer Lösungen appliziert werden.

Die Lösungen dieser Materialien bilden auf der Oberfläche der behandelten Strukturen einen Film, der je nach eingesetztem Polymertyp festigend, strukturverbessernd, formgebend, glanzverbessernd, glättend und antistatisch wirken kann. Die auf den keratinhaltigen Strukturen gebildeten Filme sollen einerseits feuchtigkeitsbeständig sein, andererseits sollen sich diese Filme bei der Reinigung leicht mit einer wäßrigen Tensidlösung entfernen lassen.

Aufgrund der ständig wachsenden Forderung nach umweltverträglicheren Erzeugnissen soll der Gehalt an flüchtigen organischen Bestandteilen (volatile organic compounds - VOC) auch in kosmetischen Zubereitungen, wie beispielsweise Haarsprays, Haargele, Schaumfestiger oder Nagellack, möglichst niedrig gehalten werden bzw. gesenkt werden. Das bedeutet, daß Alkohol als Lösungsmittel zunehmend durch Wasser ersetzt werden soll.

Bei Verwendung herkömmlicher filmbildender Polymere kann dies jedoch Schwierigkeiten bereiten, da bei höheren Wassergehalten das Auftrageverhalten der Mittel zur Behandlung keratinhaltiger Strukturen und die Trocknung der Polymerfilme nicht befriedigend sind. Sprödigkeit und verminderte Transparenz der resultierenden Filme stellen mögliche, unerwünschte Folgen dar.

Aus der US-A 3 296 204 ist die Herstellung von sulfonathaltigen Polyamiden durch Kondensation sulfonierter aromatischer Dicarbonsäuren, deren Sulfonsäuregruppen als Alkalisalz vorliegen, mit Diaminen bekannt. Die Polyamide kommen in der Textilindustrie zur Anwendung.

In der DE-C 23 08 266 wird die Verwendung sulfonierter Polyamide zur Herstellung von Polyamidgarnen beschrieben.

In der FR 2 685 001 wird die Verwendung bestimmter wasserdispergierbarer Polyamide zur Ausrüstung von Textilfasern beschrieben.

In der US 4 300 580 ist die Verwendung von sulfonatgruppenhaltigen, wasserdispergierbaren Polyestern als Haarfestiger beschrieben. Diese haben jedoch den Nachteil, nur schwer aus dem Haar auswaschbar zu sein.

Die US-A 5 158 762 betrifft wäßrige Haarsprayzusammensetzungen, in denen als Filmbildner Polymerblends aus einem wasserlöslichen Polymer und einem sulfonatgruppenhaltigen Polyester oder Polyesteramid eingesetzt werden.

Im vorliegenden Fall standen die Erfinder vor der Aufgabe, Polymere für den Einsatz als Filmbildner in Mitteln zur Behandlung keratinhaltiger Strukturen zu finden, die erhöhte Wasseranteile in den entsprechenden kosmetischen Zubereitungen ermöglichen, ohne daß anwendungstechnische Eigenschaften, wie das Auftrageverhalten, negativ beeinflußt werden. Nach der Applikation sollten außerdem nicht-klebende, klare, glatte und elastische Filme mit guter strukturverbessernder Wirkung vorliegen. Sie sollten einerseits gut haltbar, andererseits aber auch leicht entfernbar sein.

Überraschenderweise haben die Erinder festgestellt, dass sulfonatgruppentragende Polyamide, die erhältlich sind aus
A₁) 0 bis 10 mol-%, besonders bevorzugt 0 bis 5 mol-%, mindestens einer Monoaminocarbonsäure mit 2 bis 12 C-Atomen, deren Lactam oder Monoaminocarbonsäure-/-lactam-Gemischen,
A₂) 40,05 bis 50 mol-%, vorzugsweise 45 bis 50 mol-%, besonders bevorzugt 47,5 bis 50 mol-%, mindestens eines Diamins mit 2 bis 18 C-Atomen,
A₃) 0,5 bis 49,5 mol-%, vorzugsweise 5 bis 35 mol-%, besonders bevorzugt 10 bis 30 mol-%, mindestens einer sulfonatgruppentragenden Dicarbonsäure mit 4 bis 12 C-Atomen, und
A₄) 0,5 bis 49,5 mol-%, vorzugsweise 15 bis 45 mol-%, besonders bevorzugt 20 bis 40 mol-%, mindestens einer weiteren Dicarbonsäure mit 2 bis 16 C-Atomen, wobei vorzugsweise die Summe der molaren Anteile der Monomeren A₃) und A₄) dem molaren Anteil des Monomeren A₂) entspricht,
zur Lösung dieser Aufgabe geeignet sind.

Gegenstand der vorliegenden Erfindung sind daher kosmetische Mittel, insbesondere wäßrige Mittel, welche die vorstehend beschriebenen Polyamide enthalten. Vorzugsweise betrifft die vorliegende Erfindung Mittel zur Behandlung von keratinhaltigen Strukturen, insbesondere von Haar, Nägeln oder Haut.

Ein weiterer Gegenstand der vorliegenden Erfindung ist die Verwendung der erfindungsgemäßen Polyamide in der Kosmetik, vorzugsweise zur Behandlung von keratinhaltigen Strukturen, insbesondere von Haar, Nägeln oder Haut.

Die Erfindung betrifft auch ein kosmetisches Verfahren, vorzugsweise zur Behandlung von keratinhaltigen Strukturen, insbesondere von Haar, Nägeln oder Haut, bei dem die erfindungsgemmäßen Polyamide so zur Anwendung kommen, daß der gewünschte Effekt erzielt wird. Dies erfolgt im allgemeinen durch Auftragen einer wirksamen Menge der Polyamide auf die keratinhaltigen Strukturen.

Insbesondere geeignet sind Polyamide, die nur aus den Monomeren A₂), A₃) und A₄) erhältlich sind, wobei vorzugsweise der molare Anteil des Monomeren A₂ 50 mol-% und folglich die Summe der molaren Anteile der Monomeren A₃) und A₄) ebenfalls 50 mol-% beträgt.

Besonders vorteilhaft ist es, wenn mindestens zwei verschiedene Diamine zur Herstellung der erfindungsgemäßen Polymere verwendet werden.

Als Monomere A₁) eignen sich die für die Herstellung von Polyamiden bekannten Monoaminocarbonsäuren oder deren Lactame, wie beispielsweise w-Aminoundecansäure, ε-Caprolactam, Laurinlactam, Capryllactam oder Önantholactam.

Als Monomere A₂) kommen gegebenenfalls substituierte aliphatische, cycloaliphatische oder aromatische Diamine in Betracht. Geeignete Diamine sind beispielsweise Alkylendiamine oder Cycloalkyldiamine, wie 1,5-Pentandiamin, 4,4'-Diaminodicyclohexylmethan, 2,2'-(4,4'-Diaminodicyclohexyl)propan, 3,3'-Dimethyl-4,4'-diaminodicyclohexylmethan oder, vorzugsweise, Hexamethylendiamin. Weiterhin eignen sich auch Piperazin, 2,2,4-Trimethylhexamethylendiamin, 2-Butyl-2-ethyl-1,5-pentandiamin, 2-Methylpentamethylendiamin, Isophorondiamin oder 4,7-Dioxadecan-1,10-diamin.

Besonders vorteilhaft sind die verzweigten Diamine, da sie die Eigenschaften der gebildeten Filme günstig beeinflussen, z. B. durch Herabsetzung der Kristallinität.

Als sulfonatgruppentragende Monomere A₃) kommen solche in Betracht, in denen die Sulfonatgruppe als Salz vorliegt, z.B. als Salz eines Alkalimetalls, wie Lithium, Natrium oder Kalium, oder einer Ammoniumgruppe, die gegebenenfalls mit einer bis vier ali-phatischen oder aromatischen Gruppen substituiert ist. Geeignete sulfonatgruppentragende Monomere sind Salze von aliphatischen oder aromatischen Dicarbonsäuren, wie beispielsweise Sulfobernsteinsäure oder 5-Sulfopropoxyisophthalsäure. Bevorzugt wird das Natriumsalz der 5-Sulfoisophthalsäure eingesetzt.

Geeignete Monomere A₄) sind beispielsweise aliphatische Dicarbonsäuren, wie Sebacinsäure, Azelainsäure, Dodecandicarbonsäure oder bevorzugt Adipinsäure oder Sebacinsäure. Geeignete aromatische Dicarbonsäuren sind beispielsweise Isophthalsäure oder Terephthalsäure, die auch substituiert sein können, wie beispielsweise die 3-tert.-Butylisophthalsäure, weiterhin 3,3'- oder 4,4'-Diphenyldicarbonsäure, 3,3'- oder 4,4'-Diphenylmethandicarbonsäure, 3,3'- oder 4,4'-Diphenylsulfondicarbonsäure, 1,4- oder 2,6-Naphthalindicarbonsäure oder 2-Phenoxyterephthalsäure.

Die Herstellung der sulfonatgruppentragenden Polyamide kann in an sich bekannter Weise erfolgen.

Als bevorzugte Herstellweise sei der Batch-Prozeß (diskontinuierliche Herstellweise) genannt. Dabei wird die wäßrige Monomerlösung innerhalb von 0,5 bis 3 h in einem Autoklaven auf Temperaturen von 240 bis 300°C erhitzt, wobei ein Druck von 10 bis 50 bar, insbesondere 15 bis 30 bar, erreicht wird, der durch Entspannen von überschüssigem Wasserdampf bis zu 4 h konstant gehalten wird. Danach entspannt man den Autoklaven bei konstanter Temperatur innerhalb eines Zeitraumes von 0,5 und 3 h auf Normaldruck. Anschließend wird die Polymerschmelze dem Autoklaven entnommen, mit Luft oder Stickstoff abgekühlt und anschließend granuliert.

Das so erhaltene Copolyamid hat in der Regel eine Viskositätszahl zwischen 25 und 110 ml/g, bevorzugt von 30 bis 80 ml/g, gemessen an einer 0,5 gew.-%igen Lösung in 96 %iger Schwefelsäure.

Zur Herstellung der erfindungsgemäßen Polyamiddispersionen oder -lösungen können die Polyamidgranulate in 30 bis 99, vorzugsweise 60 bis 90 Gew.-%, bezogen auf die Polymermenge, Wasser durch intensives Rühren dispergiert oder gelöst werden. Die Herstellung der Dispersionen oder Lösungen erfolgt üblicherweise bei 25°C, kann aber auch bei Temperaturen bis zu 80°C durchgeführt werden. Nach Zugabe des Wassers werden die Mischungen vorzugsweise noch 0,5 bis 3 h gerührt, wobei eine vollständige Dispersion oder Lösung der Polyamide erfolgt. Die so hergestellten Dispersionen oder Lösungen weisen Feststoffgehalte von 1 bis 70, vorzugsweise 10 bis 40 Gew.-%, auf. Die Lichtdurchlässigkeit (bestimmt mittels 5 eines Vis-Spektrometers der Fa. Beckmann) liegt bei 50 bis 99 %, vorzugsweise > 60 %. Die Partikelgrößen können im Bereich von 40 bis 120 nm liegen.

Vorteilhaft an den erfindungsgemäßen sulfonatgruppentragenden Polyamiden ist, daß sie sich problemlos in Wasser zu Dispersionen oder Lösungen mit höheren Feststoffgehalten verarbeiten lassen.

Damit eignen sich die erfindungsgemäßen Polyamide vor allem für solche Mittel zur Behandlung keratinhaltiger Strukturen, die 5 einen besonders niedrigen VOC-Gehalt aufweisen sollen, also überwiegend auf Wasser basieren. Bevorzugt kann man sie in Haarfestigern mit VOC-Werten < 55 Gew.-% einsetzen. Wegen der guten Selbstdispergierbarkeit der erfindungsgemäßen Polyamide kann auf größere Anteile organischer Lösungsmittel verzichtet werden.

Jedoch eignen sich erfindungsgemäß als Filmbildner in Mitteln zur Behandlung keratinhaltiger Strukturen auch solche sulfonatgruppenhaltigen Polyamide, die durch Einsatz von geringeren oder höheren Anteilen der sulfonatgruppentragenden Monomere erhältlich sind, und zwar vor allem dann, wenn die Mittel zur Behandlung keratinhaltiger Strukturen VOC-Werte von > 55 % aufweisen können. Die Mittel zur Behandlung keratinhaltiger Strukturen stellen in einem solchen Fall alkoholische oder wäßrig-alkoholische Lösungen der Polyamide in anderen hierfür geeigneten organischen Lösungsmitteln dar. Aufgrund der besseren Verdampfbarkeit des organischen Lösungsmittels im Vergleich zu Wasser können diese Lösungen auch mit einem geringeren Feststoffgehalt, beispielsweise im Bereich von 1 bis 10 Gew.-%, als Mittel zur Behandlung keratinhaltiger Strukturen eingesetzt werden.

Erfindungsgemäß können die sulfonattragenden Polyamide sowohl als alleinige Filmbildner in Mittel zur Behandlung keratinhaltiger Strukturen als auch als Mischungen mit herkömmlichen filmbildenden Polymeren eingesetzt werden. Als herkömmliche filmbildende Polymere eignen sich beispielsweise anionische Polymere, wie Acrylsäure- oder Methacrylsäure-Homopolymere oder -Copolymere, Copolymerisate aus Acrylsäure und Acrylamiden und Copolymere auf der Basis von Alkylvinylethern und Maleinsäuremonoalkylestern, amphotere Polymere, wie Copolymerisate aus Octylacrylamid, Acrylat und Butylaminoethylmethacrylat, sowie nichtionische Polymere, wie Vinylpyrrolidon-Homopolymere, Vinylpyrrolidon-Vinylacetat-Copolymere und Copolymerisate aus Vinylpyrrolidon, Vinylacetat und Vinylpropionat. Das Verhältnis von sulfonatgruppenhaltigen Polyamiden zu den herkömmlichen filmbildenden Polymeren kann entsprechend den gewünschten anwendungstechnischen Eigenschaften gewählt werden.

Weiterhin können die Mittel zur Behandlung keratinhaltiger Strukturen übliche Hilfsstoffe, wie beispielsweise Tenside, Emulgatoren oder Duftstoffe, enthalten.

Formulierungen zur Behandlung keratinhaltiger Strukturen können beispielsweise wie folgt zusammengesetzt sein (% = Gew.-%):
1)
   - 5 %: eines Polyamids
   - 0,2 %: einer Mischung aus Parfümöl und Emulgator im Mengenver hältnis 1:3
   - 10 %: Ethanol
   - 84,8 %: Wasser
2)
   - 3,5 %: eines Polyamids
   - 1,5 %: eines Copolymers aus 60 Gew.-% N-Vinylpyrrolidon und 40 Gew.-% Vinylacetat
   - 0,2 %: Parfümöl/Emulgator
   - 94,8 %: Wasser

Anstelle eines NVP/VA-Copolymers können beispielsweise auch 1,5 % eines Terpolymers auf Acrylatbasis verwendet werden.

Bei Anwendung der erfindungsgemäßen Mittel zur Behandlung keratinhaltiger Strukturen werden klare, gut trocknende Filme erhalten, die ein angenehmes Hautgefühl vermitteln, gute Festigungswirkung und gute Entfernbarkeit zeigen.

### Beispiele 1 bis 6 (Tabelle 1)

In einem 5 l-Laborautoklaven wurden 2 kg einer Monomerenmischung der in Tabelle 1 angegebenen Zusammensetzung in 1500 ml Wasser vorgelegt. Der Autoklav wurde innerhalb 1 h auf 280°C aufgeheizt, wobei der resultierende Druck von ca. 20 bar durch Entspannen überschüssigen Wasserdampfes konstant gehalten wurde. Druck und Temperatur wurden eine weitere Stunde nicht verändert. Anschließend wurde der Autoklav unter Beibehaltung der Temperatur von 280°C innerhalb von 1 h auf Normaldruck entspannt. Anschließend wurde 2 h lang im Stickstoffstrom nachkondensiert. Die Schmelze wurde danach über eine Düse ausgefahren, in einem Luftbett gekühlt und granuliert.

Jeweils 400 g des Granulats wurden in 600 g Wasser bei Raumtemperatur unter Rühren dispergiert.

An den Granulaten wurden folgende Prüfungen durchgeführt:
a) Granulat vor der Dispergierung: Bestimmung der Viskositätszahl (VZ) in Anlehnung an DIN 53 246 (0,5 %ige Lösung des Copolyamids in 96 %iger H₂SO₄).
b) Bestimmung der Glasübergangstemperatur (Tg) und des Schmelzbereiches mittels **D**ifferential **S**canning **C**alorimetrie (DSC 5000 der Fa. Mettler) bei 20°C/min Aufheizrate.

Die Charakterisierung der Dispersionen erfolgte nach folgenden Methoden:
a) Bestimmung der Turbidität (NTU-Wert)
   Die Trübung der wäßrigen Polykondensat-Lösungen wurde durch nephelometrische Trübungsmessung bestimmt (modifizierte Methode nach DIN 38404). Bei dieser Methode wird photometrisch die Streuung von Licht nach Durchstrahlen der Meßlösung ermittelt, wobei diese Streuung durch die Wechselwirkung zwischen den Lichtstrahlen und den Partikeln in der Lösung oder Dispersion, deren Anzahl und Größe den Grad der Trübung ausmachen, bestimmt wird. Als Meßgröße dient die nephelometrische Trübungseinheit (NTU), welche bei 25°C und in 5%iger wäßriger Lösung gemessen und durch Eichung auf der Basis von Formazin als künstlichem Trübungsmittel festgelegt wird. Je höher der NTU-Wert, desto trüber ist die Lösung.
b) Bestimmung der Viskosität im Rotationsviskosimeter (Modell: RV 20; Fa. Haake) bei einer Scherung von 500 s⁻¹; T = 23°C

**Tabelle 1:**

| Zusammensetzung und Eigenschaften gemäß den Beispielen 1 bis 6 (Angaben in mol-%) | | | | | | |
|---|---|---|---|---|---|---|
| | Beispiel 1 | Beispiel 2 | Beispiel 3 | Beispiel 4 | Beispiel 5 | Beispiel 6 |
| Natrium-5-Sulfoisophthalsäure | 15,2 | 20 | 20 | 20 | 20 | 20 |
| Isophthalsäure | 24,8 | 20 | 20 | 20 | 20 | 10 |
| Hexamethylendiamin | 36 | 20 | 30 | 20 | 25 | 20 |
| AH-Salz | 20 | 20 | 20 | 15 | 20 | 20 |
| 11-Aminoundekansäure | - | - | - | 5 | 10 | - |
| 2-Methylpentamethylendiamin | - | 20 | 5 | 20 | 5 | - |
| Isophorondiamin | 4 | - | - | - | - | 10 |
| 2,2,4-Trimethyl hexamethylendiamin | - | - | 5 | - | - | 10 |
| Itaconsäure | - | - | - | - | - | 10 |
| Vz[ml/g] | 47 | 40 | 38 | 43 | 20 | 32 |
| dyn Visk [mPas] (5 % in H₂O) | 2,82 | 3,16 | 2,72 | 2,82 | 3,13 | 3,33 |
| Glastemperatur Tg [°C] | 152 | 154 | 156 | 157 | 117 | 147 |
| NTU-Wert (5 % in H₂O) | 3,7 | 1,8 | 6,4 | 7,8 | 6,8 | 10,4 |

## Patentansprüche

1. Kosmetisches Mittel, enthaltend sulfonatgruppentragende Polyamide, die erhältlich sind aus
A₁) 0 bis 10 mol-% mindestens einer Monoaminocarbonsäure mit 2 bis 12 C-Atomen, deren Lactam oder Monoaminocarbonsäure-/-lactam-Gemischen,
A₂) 40,05 bis 50 mol-% mindestens eines Diamins mit 2 bis 18 C-Atomen,
A₃) 0,5 bis 49,5 mol-% mindestens einer sulfonatgruppentragenden Dicarbonsäure mit 4 bis 12 C-Atomen, und
A₄) 0,5 bis 49,5 mol-% mindestens einer weiteren Dicarbonsäure mit 2 bis 16 C-Atomen.

2. Mittel nach Anspruch 1, worin die sulfonatgruppentragenden Polyamide erhältlich sind aus
A₁) 0 bis 10 mol-% mindestens einer Monoaminocarbonsäure mit 2 bis 12 C-Atomen, deren Lactam oder Monoaminocarbonsäure-/-lactam-Gemischen,
A₂) 45 bis 50 mol-% mindestens eines Diamins mit 2 bis 18 C-Atomen,
A₃) 5 bis 35 mol-% mindestens einer sulfonatgruppentragenden Dicarbonsäure mit 4 bis 12 C-Atomen, und
A₄) 15 bis 45 mol-% mindestens einer weiteren Dicarbonsäure mit 2 bis 16 C-Atomen.

3. Kosmetisches Mittel, enthaltend sulfonatgruppentragende Polyamide, die nur aus den Monomeren :
A₂) mindestens einem Diamin mit 2 bis 18 C-Atomen,
A₃) mindestens einer sulfonatgruppentragenden Dicarbonsäure mit 4 bis 12 C-Atomen, und
A₄) mindestens einer weiteren Dicarbonsäure mit 2 bis 16 C-Atomen,
erhältlich sind.

4. Mittel aus Anspruch 3, worin die sulfonatgruppentragenden Polyamide erhältlich sind aus
A₂) 50 mol-% mindestens eines Diamins mit 2 bis 18 C-Atomen,
A₃) 0,5 bis 49,5 mol-% mindestens einer sulfonatgruppentragenden Dicarbonsäure mit 4 bis 12 C-Atomen, und
A₄) 0,5 bis 49,5 mol-% mindestens einer weiteren Dicarbonsäure mit 2 bis 16 C-Atomen.

5. Mittel nach einem der Ansprüche 1 bis 4, worin die sulfonatgruppentragenden Polyamide aus mindestens zwei verschiedenen Diaminen erhältlich sind.

6. Mittel nach einem der Ansprüche 1 bis 5 zur Behandlung von keratinhaltigen Strukturen.

7. Mittel nach Anspruch 6 zur Behandlung von Haar, Nägeln oder Haut.

8. Mittel nach Anspruch 6 oder 7, enthaltend zusätzlich weitere filmbildende Polymere.

9. Verwendung von sulfonatgruppentragenden Polyamiden nach einem der Ansprüche 1 bis 5 als Filmbildner zur Behandlung von keratinhaltigen Strukturen.

10. Verwendung nach Anspruch 9 zur Behandlung von Haar, Nägeln oder Haut.

## Claims

1. A cosmetic composition comprising polyamides which carry sulfonate groups and are obtainable from
A₁) from 0 to 10 mol-% of at least one monoaminocarboxylic acid having 2 to 12 carbon atoms, its lactam, or monoaminocarboxylic acid/lactam mixtures,
A₂) from 40.05 to 50 mol-% of at least one diamine having 2 to 18 carbon atoms,
A₃) from 0.5 to 49.5 mol-% of at least one dicarboxylic acid having 4 to 12 carbon atoms which carries sulfonate groups, and
A₄) from 0.5 to 49.5 mol-% of at least one further dicarboxylic acid having 2 to 16 carbon atoms.

2. A composition as claimed in claim 1, wherein the polyamides which carry sulfonate groups are obtainable from
A₁) from 0 to 10 mol-% of at least one monoaminocarboxylic acid having 2 to 12 carbon atoms, its lactam, or monoaminocarboxylic acid/lactam mixtures,
A₂) from 45 to 50 mol-% of at least one diamine having 2 to 18 carbon atoms,
A₃) from 5 to 35 mol-% of at least one dicarboxylic acid having 4 to 12 carbon atoms which carries sulfonate groups, and
A₄) from 15 to 45 mol-% of at least one further dicarboxylic acid having 2 to 16 carbon atoms.

3. A cosmetic composition comprising polyamides which carry sulfonate groups, obtainable only from the following monomers:
A₂) at least one diamine having 2 to 18 carbon atoms,
A₃) at least one dicarboxylic acid having 4 to 12 carbon atoms which carries sulfonate groups, and
A₄) at least one further dicarboxylic acid having 2 to 16 carbon atoms.

4. A composition of claim 3, wherein the polyamides which carry sulfonate groups are obtainable from
A₂) 50 mol-% of at least one diamine having 2 to 18 carbon atoms,
A₃) from 0.5 to 49.5 mol-% of at least one dicarboxylic acid having 4 to 12 carbon atoms which carries sulfonate groups, and
A₄) from 0.5 to 49.5 mol-% of at least one further dicarboxylic acid having 2 to 16 carbon atoms.

5. A composition as claimed in any of claims 1 to 4, wherein the polyamides which carry sulfonate groups are obtainable from at least two different diamines.

6. A composition as claimed in any of claims 1 to 5 for treating keratin-containing structures.

7. A composition as claimed in claim 6 for treating hair, nails or skin.

8. A composition as claimed in claim 6 or 7, additionally comprising further film-forming polymers.

9. The use of polyamides which carry sulfonate groups, of any of claims 1 to 5, as film formers for treating keratin-containing structures.

10. The use as claimed in claim 9 for treating hair, nails or skin.

## Revendications

1. Produit cosmétique, contenant des polyamides portant des groupes sulfonate, qui peuvent être obtenus à partir de
A₁) 0 à 10 % en mol d'au moins un acide monoaminocarboxylique ayant 2 à 12 atomes de carbone, leur lactame ou des mélanges d'acide monoaminocarboxylique/lactame,
A₂) 40,05 à 50 % en mol d'au moins une diamine ayant 2 à 18 atomes de carbone,
A₃) 0,5 à 49,5 % en mol d'au moins un acide dicarboxylique portant des groupes sulfonate, ayant 4 à 12 atomes de carbone, et
A₄) 0,5 à 49,5 % en mol d'au moins un autre acide dicarboxylique ayant 2 à 16 atomes de carbone.

2. Produit selon la revendication 1, dans lequel les polyamides portant des groupes sulfonate peuvent être obtenus à partir de
A₁) 0 à 10 % en mol d'au moins un acide monoaminocarboxylique ayant 2 à 12 atomes de carbone, leur lactame ou des mélanges d'acide monoaminocarboxylique/lactame,
A₂) 45 à 50 % en mol d'au moins une diamine ayant 2 à 18 atomes de carbone,
A₃) 5 à 35 % en mol d'au moins un acide dicarboxylique portant des groupes sulfonate, ayant 4 à 12 atomes de carbone, et
A₄) 15 à 45 % en mol d'au moins un autre acide dicarboxylique ayant 2 à 16 atomes de carbone.

3. Produit cosmétique, contenant des polyamides portant des groupes sulfonate, qui peuvent être obtenus seulement à partir des monomères:
A₂) au moins une diamine ayant 2 à 18 atomes de carbone,
A₃) au moins un acide dicarboxylique portant des groupes sulfonate, ayant 4 à 12 atomes de carbone, et
A₄) au moins un autre acide dicarboxylique ayant 2 à 16 atomes de carbone.

4. Produit selon la revendication 3, dans lequel les polyamides contenant des groupes sulfonate peuvent être obtenus à partir de
A₂) 50 % en mol d'au moins une diamine ayant 2 à 18 atomes de carbone,
A₃) 0,5 à 49,5 % en mol d'au moins un acide dicarboxylique portant des groupes sulfonate, ayant 4 à 12 atomes de carbone, et
A₄) 0,5 à 49,5 % en mol d'au moins un autre acide dicarboxylique ayant 2 à 16 atomes de carbone.

5. Produit selon l'une des revendications 1 à 4, dans lequel les polyamides portant des groupes sulfonate peuvent être obtenus à partir d'au moins deux diamines différentes.

6. Produit selon l'une des revendications 1 à 5 pour le traitement de structures contenant de la kératine.

7. Produit selon la revendication 6 pour le traitement des cheveux, des ongles ou de la peau.

8. Produit selon la revendication 6 ou 7, contenant en outre d'autres polymères filmogènes.

9. Utilisation de polyamides portant des groupes sulfonate selon l'une des revendications 1 à 5 comme agents filmogènes pour le traitement de structures contenant de la kératine.

10. Utilisation selon la revendication 9 pour le traitement des cheveux, des ongles ou de la peau.
